# EUROPÄISCHE PATENTSCHRIFT

(11) **EP 2 129 357 B1**
(45) Veröffentlichungstag und Bekanntmachung des Hinweises auf die Patenterteilung: **09.01.2013**
(21) Anmeldenummer: 08709260.7
(22) Anmeldetag: 29.02.2008
(51) Int. Cl.: A61K 8/02, A61K 8/19, A61K 8/25, A61K 8/26, A61K 8/37, A61K 8/40, A61K 8/49, A61K 8/72, A61K 8/92, A61Q 17/04

(54) **KOSMETISCHES LICHTSCHUTZMITTEL**
COSMETIC LIGHT PROTECTIVE AGENT
PRODUIT COSMÉTIQUE PHOTOPROTECTEUR

(30) Priorität: 01.03.2007 DE 102007010861
(43) Veröffentlichungstag der Anmeldung: 09.12.2009
(73) Patentinhaber: Coty Germany GmbH, 55116 Mainz (DE)
(72) Erfinder: GOLZ-BERNER, Karin, MC-98000 Monaco (MC); ZASTROW, Leonhard, MC-98000 Monaco (MC); MITON, Sandra, F-06300 Nizza (FR)
(74) Vertreter: Gulde Hengelhaupt Ziebig & Schneider
(86) Internationale Anmeldenummer: PCT/EP2008/052507
(87) Internationale Veröffentlichungsnummer: WO 2008/104607

(56) Entgegenhaltungen:
- WO-A-2005/103659
- DE-A1- 10 253 129
- US-A1- 2005 036 961

## Beschreibung

Die Erfindung betrifft ein kosmetisches Lichtschutzmittel mit Wirkung für einen großen Wellenlängenbereich. Das Mittel zeigt eine unerwartet hohe Lichtabsorption und verhindert Hautschädigungen durch Licht der Wellenlängen 250-1400 nm weitgehend.

Es sind eine Vielzahl von Sonnenschutzmitteln mit den unterschiedlichen UVA- und UVB-Filtern bekannt. Aus WO 2006/136724 sind auch Kombinationen von UVA-, UVB- und IR-Filtern bekannt, wobei allgemein neben üblichen UV-Filtern monodisperses Siliciumdioxid oder Polystyrol sowie Acrylpolymere kombiniert werden. Die WO 03/045345 beschreibt kosmetische Zusammensetzungen mit verschiedenen weißen und farbigen Gläsern, um das UV- und IR-Verhalten sowie das Hautgefühl zu verbessern.

US 2005/036961 A1 (HANSENNE ISABELLE [US] ET AL) 17. Februar 2005 (2005-02-17) offenbart (Anspruch 1) ein kosmetisches Lichtschutzmittel, enthaltend Mikrosphären aus Glass (b), sowie eine UV-Substanz (a). Als UV-Substanz kann ([0073]) 2,2' - Methylen - bis - (6 - (2H - benzotriazol - 2 - yl) - 4 - (1,1,3,3 - tetramethylbutyl) - phenol) ausgewählt werden.

Der Erfindung liegt die Aufgabe zugrunde, ein kosmetisches Lichtschutzmittel zu entwickeln, das in einem breiten Bereich des Lichtspektrums besondere lichtschützende Eigenschaften entwickelt und damit universell eingesetzt werden kann.

Erfindungsgemäß ist das Lichtschutzmittel dadurch gekennzeichnet, dass es den Wellenlängenbereich von 250 nm bis 1400 nm abdeckt und wenigstens folgende Bestandteile umfasst
(a) als UVA-Filter wenigstens Methylene Bis-Benzotriazolyl Tetramethylbutylphenol;
(b) wenigstens eine Filtersubstanz für UVB-Strahlung;
(c) Sanddornöl mit einem Brechungsindex von 1,4 bis 1,59 und einen Feststoff mit einer Teilchengröße d₅₀ von 3 bis 50 µm, ausgewählt unter Citrin, Topas, Amethyst, Rubin, Glas mit einem Brechungsindex von 1,500 bis 1,599 bei 587,6 nm oder Gemischen davon;
(d) wenigstens ein Infrarot-absorbierendes Glas mit einer Teilchengröße d₅₀ von 5 bis 50 µm;
(e) eine radikalfangende Substanz oder ein Substanzgemisch;
wobei der Anteil der lichtschützenden Substanzen (a) bis (d) insgesamt im Bereich von 9 bis 32 Gew-% liegt, und der Rest bis 100 % neben Bestandteile(e) kosmetische Hilfs- und/oder Trägerstoffe und gegebenenfalls weitere kosmetische Wirkstoffe sind.

Es wurde gefunden, dass das erfindungsgemäße Gemisch, ausgehend vom Wellenlängenbereich 250-400 nm des UV-Bereiches über den Bereich des sichtbaren Lichtes von >400-700 nm bis in den Infrarotbereich von >700 nm bis 1400 nm eine unerwartet hohe Lichtabsorption aufweist und damit Hautschädigungen durch Licht aller dieser Wellenlängen weitgehend verhindern kann. Überraschend ist dabei, dass der zu erwartende Effekt der Einzelbestandteile des erfindungsgemäßen Gemisches deutlich von der Kombination aller Bestandteile übertroffen wird und damit einen echten Synergismus darstellt.

Besonders bevorzugt als Bestandteil (c) ist ein Gemisch aus einem optischen klaren Glas, vorzugsweise mit einer Teilchengröße d₅₀ von 3 bis 30 µm und gemahlenem Rubin oder Citrin, vorzugsweise mit einer Teilchengröße d₅₀ von 5 bis 40 µm. Das optische Glas kann ein farbiges Glas sein, z.B. ein rotes Glas. Der Bestandteil (c) kann neben dem Sanddornöl auch nur gemahlenen Rubin oder Citrin enthalten.

Unter dem Begriff "Teilchengröße d₅₀" wird eine Teilchengrößenverteilung mit Gauß'scher Verteilungskurve verstanden, bei der wenigstens 50 % der Teilchen die angegebene Größe des Teilchengrößenwertes aufweisen.

Als Filter für Infrarotstrahlung wird ein Glas eingesetzt, vorzugsweise ein grünes Glas wie Schott Nr. 8516, das einen spezifischen Transmissionsfaktor (λ = 1100 nm; d = 0,5 mm) von 16 hat und eine Teilchengröße d₅₀ von 12 µm und d₉₉ von 63 µm aus dem Glassystem SiO₂-Al₂O₃-BaO-Fe₂O₃ mit Anteilen von Sb₂O₃ und B₂O₃ sowie Li₂O. Es können jedoch auch andere IR-Gläser verwendet werden. Der Anteil an IR-Glas kann vorteilhaft im Bereich von 0,2 bis 1,6 Gew-% liegen.

Der Anteil an Methylene Bis-Benzotriazolyl Tetramethylbutylphenol (a) liegt bevorzugt im Bereich von 3 bis 8 Gew-%, insbesondere 4 bis 6 Gew-%, bezogen auf das Gesamtgewicht des Lichtschutzmittels.

Der Anteil der Filtersubstanz für UVB-Strahlung (b) liegt bevorzugt im Bereich 4 bis 20 Gew-%, insbesondere 5 bis 18 Gew-%. Dabei handelt es sich bevorzugt um ein Gemisch mehrerer Filtersubstanzen. Die Filtersubstanz für UVB-Strahlung ist beispielsweise ausgewählt aus der Gruppe umfassend Isoamyl p-Methoxycinnamate, Ethylhexyl Triazone, Ethylhexyl-Methoxycinnamate, Octocrylene, Phenylbenzimidazole Sulfonic Acid, Diethylhexyl Butamido Triazone, Ethylhexyl Salicylate und Gemischen davon.

Der Anteil an Sanddornöl liegt im Bereich von 0,1 - 1,5 Gew. %. Bei dem eingesetzten Sanddornöl handelt es sich um ein Sanddorn-Fruchtfleischöl, das durch Wirkung nativer Enzyme und Abzentrifugieren des Öls bei einer Temperatur von 10-50 °C aus den ungetrockneten Beeren gewonnen wird. Das Sanddornöl ersetzt mit dem genannten Gewichtsanteil im Lichtschutzmittel nicht weitere Fette und Öle, die die eigentliche Ölphase bilden, falls vorhanden, und weiter unten beschrieben werden.

Der Anteil an dem Feststoff, der auch ein Gemisch verschiedener Feststoffe sein kann, liegt insgesamt vorteilhaft im Bereich 0,5 bis 4 Gew-%, insbesondere 0,8 bis 2,5 Gew-%. Besonders bevorzugt ist eine Kombination von 0,1 bis 0,8 Gew-% Rubinpulver und 0,8 bis 1,8 Gew-% optischem Glas des Systems SiO₂-Na₂O-CaO-ZnO-BaO, das CuO, ZnO und MgO enthalten kann und vorteilhaft ein rotes Glas ist, z.B. Schott D 8010.

Als radikalfangende Substanzen können bekannte Antioxidationsmittel eingesetzt werden. Dazu gehören Vitamine wie Vitamin C und Derivate davon, beispielsweise Ascorbylacetat, -phosphat und -palmitat, Magnesiumascorbylphosphat; Vitamin A und Derivate davon; Folsäure und deren Derivate; Vitamin E und deren Derivate, wie Tocopherylacetat; Flavone oder Flavonoide; Aminosäuren, wie Histidin, Glycin, Tyrosin, Tryptophan und Derivate davon; Imidazole wie z.B. cis- oder trans-Urocaninsäure und deren Derivate; Peptide wie D,L-Carnosin, D-Carnosin, L-Carnosin und deren Derivate; Carotinoide und Carotine, wie z.B. α-Carotin, β-Carotin, Lycopin; Harnsäure und Derivate davon; α-Hydroxysäuren wie Citronensäure, Milchsäure, Apfelsäure; α-Hydroxyfettsäuren wie Palmitinsäure, Phytinsäure; Pflanzenextrakte und deren Gemische, wie Extrakte von Kiefernrinde, Rosmarin, Oregano, Calendula, Quebracho, Angelikawurzel, Camellia, Grünkaffee-Extrakt, Grüntee-Extrakt usw., sowie Hefeextrakte.

Ein besonders vorteilhaftes radikalfangendes Mittel ist eine Zubereitung mit einem Gehalt an einem durch Extraktion der Rinde von Quebracho blanco und nachfolgender enzymatischer Hydrolyse gewonnenen Produkt, das wenigstens 90 Gew-% Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser (z.B. WO 99/66881 Beispiel 1).

Ein weiteres vorteilhaftes radikalfangendes Mittel ist ein liposomenfreies Gemisch von Pflanzenextrakten auf alkoholischer Basis, bestehend aus dem Extrakt grüner Kaffeebohnen, dem Extrakt von Blättern von Camellia sinensis, von Pongamia pinnata und der Wurzeln von Angelica archangelica und einem einwertigen niederen Alkohol (INCI: RPF-Complex II, bzw. WO 01/26617 Beispiel 1).

Ein vorteilhaftes erfindungsgemäßes Lichtschutzmittel umfasst folgende Bestandteile:
(a) als UVA-Filter wenigstens Methylene Bis-Benzotriazolyl Tetramethylbutylphenol;
(b) eine Filtersubstanz für UVB-Strahlung ausgewählt unter Isoamyl p-Methoxycinnamate, Ethylhexyl Triazone, Ethylhexyl-Methoxycinnamate, Octocrylene, Phenylbenzimidazole Sulfonic Acid, Diethylhexyl Butamido Triazone, Ethylhexyl Salicylate und Gemischen davon;
(c) ein Gemisch aus Sanddornöl und gemahlenem Rubin oder Citrin;
(d) ein IR-Glas mit einer Teilchengröße d₅₀ von 5 bis 50 µm;
(e) eine radikalfangende Zubereitung mit einem Gehalt an einem durch Extraktion der Rinde von Quebracho blanco und nachfolgender enzymatischer Hydrolyse gewonnenem Produkt, das wenigstens 90 Gew-% Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser.

Das erfindungsgemäße Lichtschutzmittel enthält weiterhin kosmetische Hilfs- und/oder Trägerstoffe, wie sie üblicherweise in solchen Zubereitungen verwendet werden, z.B. Wasser, Konservierungsmittel, Farbstoffe, Pigmente mit färbender Wirkung, Verdickungsmittel, Duftstoffe, Alkohole, Polyole, Ester, Elektrolyte, Gelbildner, polare und unpolare Öle, Polymere, Copolymere, Emulgatoren, Wachse, Stabilisatoren, Penetrationsbeschleuniger.

Es können auch ein oder mehrere weitere kosmetische Wirkstoffe im Lichtschutzmittel enthalten sein. Dazu gehören z.B. Selbstbräunungsmittel, Feuchthaltemittel, Melanin, entzündungswidrige natürliche Wirkstoffe, mit Sauerstoff beladene asymmetrische lamellare Aggregate gemäß WO 94/00109, sowie solche zur positiven Beeinflussung der Altershaut, insbesondere in Kombination mit Biochinonen, insbesondere Ubichinon Q10, Kreatin, Kreatinin, Camitin, Biotin, Isoflavone, Cardiolipin, Liponsäure, Anti Freezing Proteine, Arctiin, Hopfen- und Hopfen-Malz-Extrakte.

In einer bevorzugten Ausführung der Erfindung enthält das Lichtschutzmittel Melanin. Dabei sind Sanddornöl und Melanin im Verhältnis 1 : 0,01 bis 0,1 enthalten, wobei der maximale Anteil von löslichem Melanin bei 0,1 Gew-% liegen kann, vorzugsweise 0,03 Gew-%, bezogen auf das Gesamtgewicht des Lichtschutzmittels.

Auch Wirkstoffe zur Unterstützung der Hautfunktionen bei trockener Haut können zugesetzt werden, wie insbesondere Vitamin C, Biotin, Camitin, Kreatin, Propionsäure, Grüntee-Extrakte, Eucalyptusöl, Harnstoff und Mineralsalze, insbesondere NaCl, Meeresmineralien sowie Osmolyte.

Auch Wirkstoffe zur Linderung und/oder positiven Beeinflussung von irritativen Hautzuständen sind verwendbar, insbesondere Sericoside, Extrakte des Süßholzes, Licochalcone, insbesondere Licochalcone A, Silymarin, Silyphos, Dexpan-thenol.

Pigmentierungsbeeiflussende Wirkstoffe, die die erfindungsgemäßen Lichtschutzmittel zusätzlich enthalten können, sind insbesondere Tyrosinsulfat, 8-Hexadecen-1,16-dicarbonsäure, Liponsäure, Liponamid, Extrakte des Süßholzes, Kojisäure, Hydrochinon, Arbutin, Fruchtsäuren, insbesondere α-Hydroxy-Säuren (AHAs), Uvae ursi, Ursolsäure, Ascorbinsäure, Grüntee-Extrakte, Aminoguanidin, Pyridoxamin. Dazu gehören auch solche Substanzen, die eine verstärkte oder schnellere Bräunung der Haut herbeiführen, insbesondere Lipofuscine, Purine, Pyrimidine, Dihydroxyaceton, Erythrulose.

Die erfindungsgemäßen Lichtschutzmittel enthalten in jedem Fall wenigstens einen weiteren UVB-Filter. Es können jedoch zusätzlich mehrere wasser- und/oder öllösliche UVA- oder UVB-Filter oder beide zugesetzt werden. "UVB-Filter" im Sinne der Erfindung sind auch so genannte Breitbandfilter.

Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxy-zimtsäure(2-ethylhexyl)ester; Benzophenon-Derivate wie 2-Hydroxy-4-methoxybenzophenon; 3-Benzylidencampher-Derivate wie 3-Benzylidencampher.

Bevorzugte öllösliche UV Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern, die zusammen mit Methylene Bis-Benzotriazolyl Tetramethylbutylphenol eingesetzt werden können, gehören Benzophenone-3, Dibenzoylmethan-Derivate wie 1-Phenyl-4-(4'-isopropylphenyl)propan-1,3-dion, Butyl Methoxybenzoyl-methane oder Menthyl Anthranilate sowie Disodium Phenyl Dibenzylmadazole Tetrasulfonate und Diethylamino Hydroxybenzoyl Hexylbenzoate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Disodium Phenyl Dibenzimidazole Tetrasulfonate, Bis-Ethylhexyloxyphenol Methoxyphenyl Triazine.

Weiterhin können auch Breitbandfilter eingesetzt werden, wie z.B. Bis-Resorcinyltriazin-Derivate, oder auch Benzoxazole.

Weiterhin einsetzbar als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch verwendet werden können.

Besonders bevorzugt als anorganische Filter sind agglomerierte Substrate von TiO₂ und/oder ZnO, die einen Gehalt an sphärischen und porösen SiO₂-Teilchen aufweisen, wobei die SiO₂-Teilchen eine Teilchengröße im Bereich von 0,05 µm bis 1,5 µm haben, und neben den SiO₂-Teilchen andere anorganische teilchenförmige Stoffe mit sphärischer Struktur vorliegen, wobei die sphärischen SiO₂-Teilchen mit den anderen anorganischen Stoffen definierte Agglomerate mit einer Teilchengröße im Bereich von 0,06 µm bis 5 µm bilden (gemäß WO 99/06012).

Besonders geeignete Öle für die erfindungsgemäßen Lichtschutzmittel sind beispielsweise Siliconöle, Mineralöle, Hydrogenated Polyisobuten, Polyisopren, Squalane, Tridecyltrimellitat, Trimethylpropantriisostearat, Isodecylcitrat, Neopentylglycol-diheptanoat, PPG-15-stearylether sowie pflanzliche Öle, wie Calendulaöl, Jojobaöl, Avocadoöl, Macadamianussöl, Rizinusöl, Kakaobutter, Kokosnussöl, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinussöl und Gemische davon, sowie kosmetische Ester oder Ether, die verzweigt oder unverzweigt, gesättigt oder ungesättigt sein können oder Gemische zweier oder mehrerer dieser Öle, Ester oder Ether.

Das erfindungsgemäße Mittel kann auch übliche Feuchthaltemittel enthalten, wie Glycerin, Butylenglycol, Propylenglycol und Gemische davon.

Da das erfindungsgemäße Lichtschutzmittel als Emulsion oder Gel zubereitet werden kann, können auch für Gele geeignete Gelbildner eingesetzt werden. Dazu gehören z.B. Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon, Montmorillonit.

Geeignete Emulgatoren für O/W-Emulsionen sind beispielsweise Anlagerungsprodukte von 2-30 Mol Ethylenoxid an lineare C₈-C₂₂-Fettalkohole, an C₁₂-C₂₂-Fettsäuren und an C₈-C₁₅-Alkylphenole; C₁₂-C₂₂-Fettsäuremono- und -diester von Anlagerungsprodukten von 1-30 Mol Ethylenoxid an Glycerin. Geeignete Emulgatoren für W/O-Emulsionen sind beispielsweise Anlagerungsprodukte von 2-15 Mol Ethylenoxid an Ricinusöl; Ester von C₁₂-C₂₂-Fettsäuren und Glycerin, Polyglycerin, Pentaerythrit, Zuckeralkohole (z.B. Sorbit), Polyglucoside (z.B. Cellulose); Polyalkylenglycole; Wollwachsalkohole; Copolymere von Polysiloxan-Polyalkylpolyether.

Ein Zusatz von Elektrolyten bewirkt eine Veränderung des Löslichkeitsverhaltens eines hydrophilen Emulgators. Hydrophile Emulgatoren unterliegen einer partiellen Phaseninversion, bei der eine Solubilisierung von Wasser durch die Ölphase auftritt. Dabei resultiert eine stabile Emulsion, insbesondere eine Mikroemulsion oder auch eine O/W/O-Emulsion. Geeignete Elektrolyte sind Salze mit den folgenden Anionen: Chloride, anorganische Oxo-Element-Anionen, wie Borate, Aluminate, Sulfate Phosphate, Carbonate. Zu den auf organischen Anionen basierenden Elektrolyten gehören Citrate, Tartrate, Lactate, Propionate, Acetate und Benzoate sowie EDTA und deren Salze.

Kationen der Salze können sein Alkalimetall-, Erdalkalimetall-, Ammonium-, Alkylammonium-, Eisen-, ZinkIonen.

Die Verwendung des erfindungsgemäßen Lichtschutzmittels kann z.B. erfolgen in Sonnencremes, Sonnengelen, Sonnensprays, Aerosole, After-sun-Produkten, Tagescremes, Nachtcremes, Masken, Körperlotionen, Make up's, Lippenstiften, Lippenbalsam, Augenkosmetik, Haarmasken, Haarshampoos, Duschgelen, Duschölen, Badeölen. Die Herstellung derartiger Produkte erfolgt auf eine Weise, wie sie dem Fachmann auf diesem Gebiet bekannt ist.

Die Erfindung soll nachstehend durch Beispiele näher erläutert werden. Alle Angaben erfolgen in Gewichtsprozent, sofern nichts anderes angegeben ist.

### Beispiel 1 Lichtschutzcreme 1

| **Phase A** | |
|---|---|
| Wasser | q.s. ad 100 |
| Xanthan Gum | 0,25 |
| Propylene Glycol | 2 |
| Acrylates/Vinyl Isodecanoate Crosspolymer | 0,3 |
| Cyclopentasiloxan & Cyclohexasiloxane & | |
| Cyclotetrasiloxane | 3 |
| Tetrasodium EDTA | 0,1 |
| Sodium Hydroxide | 0,1 |

| **Phase B** | |
|---|---|
| Isoamyl p-Methoxycinnamate | 5 |
| Octocrylene | 3 |
| Butyl Methoxydibenzoylmethane | 4 |
| Bis-Ethylhexyloxyphenol Methoxyphenyl Triazine | 1 |
| Methylene Bis-Benzotriazolyl Tetramethylbutylphenol | 5 |
| Dicaprylyl Carbonate & Tocopherol | 6 |

| **Phase C** | |
|---|---|
| RPF Complex I¹ | 1 |
| Hippophae Rhamnoides | 0,5 |
| Glass (Schott D8010) | 3,0 |
| Ruby Powder | 0,1 |
| Glass (Schott 8516) | 1,5 |
| Ethanol | 3 |
| Konservierungsmittel | 0,9 |
| Parfümöl | 0,75 |

| | |
|---|---|
| ¹ gemäß WO 99/66881 Wirkstoffkomplex von Beispiel 1 | |

Die Phasen A und B werden separat hergestellt und bei 75 °C zusammengegeben und unter Rühren sorgfältig vermischt. Nach dem Abkühlen des Gemisches erfolgt unter Rühren bei 45 °C die Zugabe der Phase C.

### Beispiel 2 Lichtschutzcreme 2

| **Phase A** | |
|---|---|
| Wasser | q.s. ad 100 |
| Xanthan Gum | 0,25 |
| Propylene Glycol | 2 |
| Acrylates/Vinyl Isodecanoate Crosspolymer | 0,3 |
| Cyclopentasiloxan & Cyclohexasiloxane & | |
| Cyclotetrasiloxane | 3-5 |
| Tetrasodium EDTA | 0,1 |
| Sodium Hydroxide | 0,1 |

| **Phase B** | |
|---|---|
| Isoamyl p-Methoxycinnamate | 4-5 |
| Octocrylene | 2 |
| Butyl Methoxydibenzoylmethane | 3 |
| Bis-Ethylhexyloxyphenol Methoxyphenyl Triazine | 1 |
| Methylene Bis-Benzotriazolyl Tetramethylbutylphenol | 4 |
| Dicaprylyl Carbonate & Tocopherol | 5 |

| **Phase C** | |
|---|---|
| RPF Complex I¹ | 0,5 |
| Hippophae Rhamnoides | 0,3 |
| Glass (Schott D8010) | 1,0 |
| Ruby Powder | 0,2 |
| Glass (Schott 8516) | 0,5 |
| Melanin Soluble | 0,01 |
| Ethanol | 3 |
| Konservierungsmittel | 0,9 |
| Parfümöl | 0,75 |

| | |
|---|---|
| ¹ gemäß WO 99/66881 Wirkstoffkomplex von Beispiel 1 Die Herstellung erfolgt wie im Beispiel 1. | |

### Beispiel 3 Vergleichsbeispiel

Es ist bekannt, dass durch UVB-Strahlung in der Haut freie Radikale gebildet werden, die bei entsprechender Dauer der Strahlungseinwirkung zu Erythemen der Haut führen. Darauf basiert u.a. die Angabe des so genannten Licht- oder Sonnenschutzfaktors LSF bzw. SPF (Sun Protection Factor), mit denen kosmetische Sonnenschutzprodukte deklariert sind.

Es ist weiterhin bekannt, dass bei Einbeziehung der UVA-Strahlung zusätzlich zur UVB-Strahlung eine starke Erhöhung der Zahl freier Radikale sowie eine Verstärkung der Wirkung freier Radikale in der Haut auftritt. Dazu wurde ein so genannter Integraler Sonnenschutzfaktor (ISPF) vorgeschlagen (WO 2005/103659)

Es wurde nun gefunden, dass auch die Strahlung im sichtbaren Bereich sowie Infrarotstrahlung freie Radikale in der Haut hervorrufen kann, so dass der Integrale Sonnenschutzfaktor sinnvoll über den gesamten Bereich der Wellenlängen von 250 bis 1400 nm zu erweitern wäre.

Von dieser Überlegung ausgehend, die noch eine Reihe weiterer Untersuchungen erforderlich macht, sind vorab Messungen von freien Radikalen mit der in der WO 2005/103659 genannten Methodik (Hautbiopsien) in verschiedenen Wellenlängenbereichen vorgenommen und mit Einsatz der Wirksubstanzen als ISPF ausgedrückt worden.

Dabei zeigen die Wirksubstanzen von Beispiel 1 folgendes Ergebnis als jeweilige Gruppe für ihren jeweiligen Wellenlängenbereich:
18% UVA/UVB-Filter¹ für den Bereich 200-400 nm = ISPF_{UVA/B} = 7,6
1,5 % Filter² für den Bereich 400-700 nm = ISPF_{VIS} = 2,0
0,5 % Filter³ für den Bereich 700-1400 nm = ISPF_{IR} = 1,3

¹ Isoamyl p-Methoxycinnamate, Octocrylene, Butyl Methoxydibenzoylmethane, Bis-Ethylhexyloxyphenol Methoxyphenyl Triazine, Methylene Bis-Benzotriazolyl Tetramethylbutylphenol

² Hippophae Rhamnoides, Glass (Schott D6010), Ruby Powder

³ Glass (Schott 8516)

Daraus wäre ein theoretischer Gesamt-ISPF von 7,6+2+1,3 = 10,9 zu erwarten.

Bei der Ermittlung des ISPF für das Gesamtgemisch aller Filter wurde jedoch ein praktischer Gesamt-ISPF von 13,9 gefunden.

Dies zeigt einen unerwartet hohen Effekt des Gemisches aller Wirksubstanzen gegenüber den Einzelgruppen, der sich aus der Kombination der Einzelbestandteile und ihrer im einzelnen bekannten Wirksamkeit nicht ableiten lässt. Dieser Synergismus unterstreicht sowohl die Bedeutung der Erfindung an sich als auch die Bedeutung der Erfassung aller freier Radikale im genannten Wellenlängenbereich und deren Berücksichtigung bei der Bereitstellung neuer Lichtschutzmittel.

## Patentansprüche

1. Kosmetisches Lichtschutzmittel, **dadurch gekennzeichnet, dass** es den Wellenlängenbereich von 250 nm bis 1400 nm erfasst und wenigstens folgende Bestandteile umfasst
(a) als UVA-Filter wenigstens Methylene Bis-Benzotriazolyl Tetramethylbutylphenol;
(b) wenigstens eine Filtersubstanz für UVB-Strahlung;
(c) Sanddornöl mit einem Brechungsindex von 1,4 bis 1,59 und einen Feststoff mit einer Teilchengröße d₅₀ von 3 bis 50 µm, ausgewählt unter Citrin, Topas, Amethyst, Rubin, Glas mit einem Brechungsindex von 1,500 bis 1,599 bei 587,6 nm oder Gemischen davon;
(d) wenigstens ein Infrarot-absorbierendes Glas mit einer Teilchengröße d₅₀ von 5 bis 50 µm;
(e) eine radikalfangende Substanz oder ein Substanzgemisch;
wobei der Anteil der lichtschützenden Substanzen (a) bis (d) insgesamt im Bereich von 9 bis 32 Gew-% liegt, und der Rest bis 100 % neben Bestandteil (e) kosmetische Hilfsstoffe, Trägerstoffe und/oder weitere kosmetische Wirkstoffe sind.

2. Lichtschutzmittel nach Anspruch 1, **dadurch gekennzeichnet, dass** das Mittel als weiteren kosmetischen Wirkstoff Melanin enthält.

3. Lichtschutzmittel nach Anspruch 1 oder 2, **dadurch gekennzeichnet, dass** die Substanz (c) ein Gemisch aus Sanddornöl und einem gemahlenen, klaren Glas mit einem Brechungsindex von 1,50 bis 1,59 und einem gemahlenen Rubin ist.

4. Lichtschutzmittel nach einem der Ansprüche 1 bis 3, , **dadurch gekennzeichnet, dass** es folgende Bestandteile umfasst
(a) als UVA-Filter wenigstens Methylene Bis-Benzotriazolyl Tetramethylbutylphenol;
(b) eine Filtersubstanz für UVB-Strahlung ausgewählt unter Isoamyl p-Methoxycinnamate, Ethylhexyl Triazone, Ethylhexyl-Methoxycinnamate, Octocrylene, Phenylbenzimidazole Sulfonic Acid, • Diethylhexyl Butamido Triazone, Ethylhexyl Salicylate und Gemischen davon;
(c) ein Gemisch aus Sanddornöl und gemahlenem Rubin oder Citrin;
(d) ein IR-Glas mit einer Teilchengröße d₅₀ von 5 bis 50 µm;
(e) eine radikalfangende Zubereitung mit einem Gehalt an einem durch Extraktion der Rinde von Quebracho blanco und nachfolgender enzymatischer Hydrolyse gewonnenem Produkt, das wenigstens 90 Gew-% Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser.

## Claims

1. A light-protective agent, **characterized in that** it covers the wavelength range of from 250 nm to 1400 nm and comprises at least the following ingredients:
(a) at least methylene bis-benzotriazolyl tetramethylbutylphenol as UVA filter;
(b) at least one filter substance for UVB radiation;
(c) sea buckthorn oil with a refractive index of from 1.4 to 1.59 and a solid with a particle size d₅₀ of from 3 to 50 µm, selected among citrine, topaz, amethyst, ruby, glass with a refractive index of from 1.500 to 1.599 at 587.6 nm or mixtures thereof;
(d) at least one infrared-absorbing glass with a particle size d₅₀ of from 5 to 50 µm;
(e) a free-radical scavenging substance or mixture of substances;
wherein the total share of light-protective substances (a) through (d) ranges from 9 to 32 wt.%, and the balance to make 100%, apart from ingredient (e), are cosmetic auxiliary substances, carrier substances and/or excipients further cosmetic active substances.

2. The light-protective agent according to claim 1, **characterized in that** the agent includes melanin as additional cosmetic active substance.

3. The light-protective agent according to claim 1 or 2, **characterized in that** substance (c) is a mixture of sea buckthorn oil and a ground clear glass with a refractive index of from 1.50 to 1.59 and a ground ruby.

4. The light-protective agent according to any one of claims 1 to 3, **characterized in that** it comprises the following ingredients:
(a) at least methylene bis-benzotriazolyl tetramethylbutylphenol as UVA filter;
(b) a filter substance for UVB radiation selected among isoamyl p-methoxycinnamate, ethylhexyl triazone, ethylhexyl methoxycinnamate, octocrylene, phenylbenzimidazole sulfonic acid, diethylhexyl butamido triazone, ethylhexyl salicylate and mixtures thereof;
(c) a mixture of sea buckthorn oil and ground ruby or citrine;
(d) an IR glass with a particle size d₅₀ of from 5 to 50 µm;
(e) a free-radical scavenging preparation having a content of a product obtained by extraction of the bark of Quebracho blanco and subsequent enzymatic hydrolysis, which product includes at least 90 wt.% of proanthocyanidin oligomers and at most 10 wt.% of gallic acid, in microcapsules, and a silkworm extract obtained by extraction, which includes the peptide cecropin, amino acids and a vitamin mixture, and a non-ionic, cationic or anionic hydrogel or mixture of hydrogels, and one or more phospholipids, and water.

## Revendications

1. Produit cosmétique protecteur de lumière, **caractérisé en ce qu'**il couvre la gamme des longueurs d'ondes comprise entre 250 nm et 1400 nm et comprend au moins les constituants suivants
(a) en tant que filtre UV-A au moins le méthylène bis-benzotriazolyl tétraméthylbutylphénol ;
(b) au moins une substance filtrante destinée au rayonnement UV-B ;
(c) de l'huile d'argousier ayant un indice de réfraction compris entre 1,4 et 1,59 et une matière solide ayant une taille des particules d₅₀ comprise entre 3 et 50 µm, choisie parmi la citrine, la topaze, l'améthyste, le rubis, le verre ayant un indice de réfraction compris entre 1,500 et 1,599 à 587,6 nm ou leurs mélanges ;
(d) au moins un verre absorbant les infrarouges, ayant une taille des particules d₅₀ comprise entre 5 et 50 µm ;
(e) une substance neutralisant les radicaux ou un mélange de substances ;
la proportion globale des substances protectrices de lumière (a) à (d) étant comprise entre 9 et 32 % en poids, et le complément pour atteindre 100 % étant, à part le constituant (e), des agents auxiliaires cosmétiques, des charges et/ou d'autres principes actifs cosmétiques.

2. Produit protecteur de lumière selon la revendication 1, **caractérisé en ce que** ledit produit contient de la mélanine en tant qu'autre principe actif cosmétique.

3. Produit protecteur de lumière selon la revendication 1 ou 2, **caractérisé en ce que** la substance (c) est un mélange d'huile d'argousier et d'un verre transparent broyé ayant un indice de réfraction compris entre 1,50 et 1,59 et d'un rubis broyé.

4. Produit protecteur de lumière selon l'une des revendications 1 à 3, **caractérisé en ce qu'**il comprend les constituants suivants
(a) en tant que filtre UV-A au moins le méthylène bis-benzotriazolyl tétraméthylbutylphénol
(b) une substance filtrante destinée au rayonnement UV-B, choisie parmi le p-méthoxycinnamate d'isoamyle, la éthylhexyl triazone, le méthoxycinnamate d'éthylhexyle, l'octocrylène, l'acide phénylbenzimidazole sulfonique, la diéthylhexyl butamido triazone, le salicylate d'éthylhexyle et leurs mélanges ;
(c) un mélange d'huile d'argousier et de rubis ou de citrine broyé(e) ;
(d) un verre IR ayant une taille des particules d₅₀ comprise entre 5 et 50 µm ;
(e) une préparation neutralisant les radicaux, contenant un produit obtenu par extraction de l'écorce de Quebracho blanco suivie d'une hydrolyse enzymatique, ledit produit contenant au moins 90 % en poids d'oligomères de proanthocyanidine et au maximum 10 % en poids d'acide gallique, dans des microcapsules, ainsi qu'un extrait de vers à soie obtenu par extraction lequel contient le peptide cécropine, des acides aminés et un mélange de vitamines, et un hydrogel non-ionique, cationique ou anioniques ou un mélange d'hydrogels, et un ou plusieurs phospholipides, et de l'eau.
